# FASCICULE DE BREVET EUROPEEN

(11) **EP 3 655 014 B1**
(45) Date de publication et mention de la délivrance du brevet: **16.06.2021**
(21) Numéro de dépôt: 18752824.5
(22) Date de dépôt: 20.07.2018
(51) Int. Cl.: A61K 38/17, A61P 9/10, G01N 33/92

(54) **CLUSTERINE POUR SON UTILISATION DANS LE TRAITEMENT DES MICRO-ANGIOPATHIES THROMBOTIQUES**
CLUSTERIN ZUR VERWENDUNG BEI DER BEHANDLUNG VON THROMBOTISCHEN MIKROANGIOPATHIEN
CLUSTERIN FOR USE IN THE TREATMENT OF THROMBOTIC MICROANGIOPATHIES

(30) Priorité: 21.07.2017 FR 1756912
(43) Date de publication de la demande: 27.05.2020
(73) Titulaire: Université de Bordeaux, 33000 Bordeaux (FR); CENTRE NATIONAL DE LA RECHERCHE SCIENTIFIQUE, 75016 Paris (FR); Centre Hospitalier Universitaire de Bordeaux, 33400 Talence (FR); INSERM - Institut National de la Santé et de la Recherche Médicale, 75013 Paris (FR); Université d'Angers, 49100 Angers (FR); Centre Hospitalier Universitaire D'angers, 49000 Angers (FR)
(72) Inventeur: AUGUSTO, Jean-François, 49140 Soucelles (FR); CONTIN-BORDES, Cécile, 33700 Mérignac (FR); DELMAS, Yahsou, 33700 Mérignac (FR); BLANCO, Patrick, 33490 Verdelais (FR); DELNESTE, Yves, 49080 Bouchemaine (FR); JEANNIN, Pascale, 49080 Bouchemaine (FR); BEAUVILLAIN, Céline, 49330 Les Hauts D'Anjou (FR)
(74) Mandataire: Novagraaf Technologies
(86) Numéro de dépôt international: PCT/FR2018/051854
(87) Numéro de publication internationale: WO 2019/016485

(56) Documents cités:
- WO-A1-2014/035876
- WO-A1-2016/176565
- WO-A2-02/42441
- WO-A2-2013/188686
- DATABASE BIOSIS [Online] BIOSCIENCES INFORMATION SERVICE, PHILADELPHIA, PA, US; mars 2001 (2001-03), YASUDA KAZUE: "Terminal complement complex (TCC) levels in urine in patients with renal diseases", XP055458171, Database accession no. PREV200100337654 & HOKKAIDO JOURNAL OF MEDICAL SCIENCE, vol. 76, no. 2, mars 2001 (2001-03), pages 71-84, ISSN: 0367-6102
- CRAGGS L J L ET AL: "Clusterin/Apolipoprotein J immunoreactivity is associated with white matter damage in cerebral small vessel diseases", NEUROPATHOLOGY AND APPLIED NEUROBIOLOGY, vol. 42, no. 2, février 2016 (2016-02), pages 194-209, XP055458162,
- COFIELL ROXANNE ET AL: "Eculizumab reduces complement activation, inflammation, endothelial damage, thrombosis, and renal injury markers in aHUS", BLOOD, vol. 125, no. 21, mai 2015 (2015-05), pages 3253-3262, XP055458166,
- CUNIN P ET AL: "Clusterin facilitates apoptotic cell clearance and prevents apoptotic cell-induced autoimmune responses", CELL DEATH & DISEASE, vol. 7, mai 2016 (2016-05), XP055458164,
- BENZ KERSTIN ET AL: "Thrombotic microangiopathy: new insights.", CURRENT OPINION IN NEPHROLOGY AND HYPERTENSION MAY 2010, vol. 19, no. 3, mai 2010 (2010-05), pages 242-247, XP055458146, ISSN: 1473-6543
- LANKFORD ET AL: "Thrombotic thrombocytopenic purpura: New insights in disease pathogenesis and therapy", TRANSFUSION MEDICINE REV, GRUNE AND STRATTON, ORLANDO, FL, US, vol. 14, no. 3, 1 juillet 2000 (2000-07-01), pages 244-257, XP005418311, ISSN: 0887-7963, DOI: 10.1053/TM.2000.7394

### SEQUENCE LISTING

<110> UNIVERSITE DE BORDEAUX CENTRE NATIONAL DE LA RECHERCHE SCIENTIFIQUE CENTRE HOSPITALIER UNIVERSITAIRE DE BORDEAUX INSTITUT NATIONAL DE LA SANTE ET DE LA RECHERCHE MEDICALE UNIVERSITE D'ANGERS CENTRE HOSPITALIER UNIVERSITAIRE D'ANGERS
<120> CLUSTERINE POUR SON UTILISATION DANS LE TRAITEMENT DES MICRO-ANGIOPATHIES THROMBOTIQUES
<130> BNT222879PC00
<150> FR1756912
   <151> 2017-07-21
<160> 1
<170> PatentIn version 3.5
<210> 1
   <211> 449
   <212> PRT
   <213> Homo sapiens
<400> 1
   Met Met Lys Thr Leu Leu Leu Phe Val Gly Leu Leu Leu Thr Trp Glu
   1               5                   10                  15
   Ser Gly Gln Val Leu Gly Asp Gln Thr Val Ser Asp Asn Glu Leu Gln
               20                  25                  30
   Glu Met Ser Asn Gln Gly Ser Lys Tyr Val Asn Lys Glu Ile Gln Asn
           35                  40                  45
   Ala Val Asn Gly Val Lys Gln Ile Lys Thr Leu Ile Glu Lys Thr Asn
       50                  55                  60
   Glu Glu Arg Lys Thr Leu Leu Ser Asn Leu Glu Glu Ala Lys Lys Lys
   65                  70                  75                  80
   Lys Glu Asp Ala Leu Asn Glu Thr Arg Glu Ser Glu Thr Lys Leu Lys
                   85                  90                  95
   Glu Leu Pro Gly Val Cys Asn Glu Thr Met Met Ala Leu Trp Glu Glu
               100                 105                 110
   Cys Lys Pro Cys Leu Lys Gln Thr Cys Met Lys Phe Tyr Ala Arg Val
           115                 120                 125
   Cys Arg Ser Gly Ser Gly Leu Val Gly Arg Gln Leu Glu Glu Phe Leu
       130                 135                 140
   Asn Gln Ser Ser Pro Phe Tyr Phe Trp Met Asn Gly Asp Arg Ile Asp
   145                 150                 155                 160
   Ser Leu Leu Glu Asn Asp Arg Gln Gln Thr His Met Leu Asp Val Met
                   165                 170                 175
   Gln Asp His Phe Ser Arg Ala Ser Ser Ile Ile Asp Glu Leu Phe Gln
               180                 185                 190
   Asp Arg Phe Phe Thr Arg Glu Pro Gln Asp Thr Tyr His Tyr Leu Pro
           195                 200                 205
   Phe Ser Leu Pro His Arg Arg Pro His Phe Phe Phe Pro Lys Ser Arg
       210                 215                 220
   Ile Val Arg Ser Leu Met Pro Phe Ser Pro Tyr Glu Pro Leu Asn Phe
   225                 230                 235                 240
   His Ala Met Phe Gln Pro Phe Leu Glu Met Ile His Glu Ala Gln Gln
                   245                 250                 255
   Ala Met Asp Ile His Phe His Ser Pro Ala Phe Gln His Pro Pro Thr
               260                 265                 270
   Glu Phe Ile Arg Glu Gly Asp Asp Asp Arg Thr Val Cys Arg Glu Ile
           275                 280                 285
   Arg His Asn Ser Thr Gly Cys Leu Arg Met Lys Asp Gln Cys Asp Lys
       290                 295                 300
   Cys Arg Glu Ile Leu Ser Val Asp Cys Ser Thr Asn Asn Pro Ser Gln
   305                 310                 315                 320
   Ala Lys Leu Arg Arg Glu Leu Asp Glu Ser Leu Gln Val Ala Glu Arg
                   325                 330                 335
   Leu Thr Arg Lys Tyr Asn Glu Leu Leu Lys Ser Tyr Gln Trp Lys Met
               340                 345                 350
   Leu Asn Thr Ser Ser Leu Leu Glu Gln Leu Asn Glu Gln Phe Asn Trp
           355                 360                 365
   Val Ser Arg Leu Ala Asn Leu Thr Gln Gly Glu Asp Gln Tyr Tyr Leu
       370                 375                 380
   Arg Val Thr Thr Val Ala Ser His Thr Ser Asp Ser Asp Val Pro Ser
   385                 390                 395                 400
   Gly Val Thr Glu Val Val Val Lys Leu Phe Asp Ser Asp Pro Ile Thr
                   405                 410                 415
   Val Thr Val Pro Val Glu Val Ser Arg Lys Asn Pro Lys Phe Met Glu
               420                 425                 430
   Thr Val Ala Glu Lys Ala Leu Gln Glu Tyr Arg Lys Lys His Arg Glu
           435                 440                 445
   Glu

## Description

### Domaine technique

La présente invention se rapporte à l'utilisation d'une protéine pour son utilisation dans le traitement des micro-angiopathies thrombotiques, ainsi qu'à un procédé *ex vivo* de stratification d'un patient atteint de MAT ou susceptible de l'être.

### État de la technique

La dénomination de microangiopathie thrombotique (MAT) regroupe un ensemble de maladies rares caractérisées par la survenue de lésions endothéliales qui aboutissent à l'occlusion des vaisseaux de petit calibre par thrombose et qui ont pour conséquence une ischémie tissulaire. L'ischémie tissulaire est responsable d'une destruction cellulaire et de la libération dans le milieu extracellulaire par les cellules mourantes de molécules normalement confinées dans la cellule. Ces molécules de danger sont pro-inflammatoires et ont une action cytotoxique pour les cellules de l'hôte. Des données récentes identifient les histones (sous forme libre ou complexées à l'ADN) comme des molécules de danger dont le rôle est central au cours des MAT.

Les MAT sont des pathologies qui engagent le pronostic vital, le rein et le cerveau étant les organes cibles les plus fréquemment atteints. Les formes les plus classiques de MAT sont le purpura thrombotique thrombocytopénique (PTT), et le syndrome hémolytique et urémique (SHU), qui peut être soit associé à des bactéries, soit non associé à des bactéries. Le SHU associé à des bactéries est la forme de SHU la plus fréquente (90% des SHU); plusieurs souches de bactéries (Escherichia coli O157:H7 ; shigelles, pneumocoques, Escherichia coli O104 notamment) sont actuellement connues pour provoquer la maladie en sécrétant une toxine appelée shiga toxine. Le SHU non associé à des bactéries représente environ 10% des SHU où il est retrouvé fréquemment en contexte de susceptibilité génétique.

Le nombre de nouveaux cas de PTT est de 5 à 10 cas par million d'habitants et par an. La fréquence des SHU est également très proche de ces valeurs.

Les MAT incluent, en plus de ces formes les plus classiques, le syndrome de HELLP, qui est une complication grave ou une variante de pré-éclampsie chez la femme enceinte, caractérisé par une hémolyse, une augmentation des enzymes hépatiques et une numération plaquettaire faible. Il existe également des formes secondaires de MAT qui surviennent dans un contexte spécifique comme par exemple lors de certains traitements, lors d'une chimiothérapie, lors de cancers ou lors d'une allogreffe de moelle.

La prise en charge actuelle des formes les plus classiques se fait généralement par échanges plasmatiques au début de la prise en charge, à l'exception du SHU associé à une shiga toxine survenant chez les enfants. Les échanges plasmatiques visent à apporter de grands volumes de plasma issu de donneurs volontaires et qui contiennent la protéine ADAMTS13 (dans le PTT) ou des protéines du complément (dans le SHU). D'autres traitements y sont souvent associés, en particulier des corticostéroïdes. Quand ils sont efficaces, les échanges plasmatiques sont poursuivis jusqu'à une normalisation durable du taux de plaquettes (au moins 48 heures) puis ils sont progressivement espacés. Les échanges plasmatiques se montrent toutefois dans de nombreux cas peu efficaces.

Dans les situations où la réponse au traitement standard n'est pas optimale, des traitements complémentaires sont ajoutés. Le traitement le plus communément utilisé dans ce contexte, un anticorps anti-CD20 (Rituximab), vise à lutter contre la production d'anticorps par le patient en détruisant transitoirement les lymphocytes B. Son utilisation se fait le plus souvent dans le cadre de protocoles thérapeutiques et en concertation avec le centre de référence.

Chez les patients atteints de SHU idiopathique dit atypique, le traitement comporte des médicaments capables de bloquer les protéines du complément à l'origine de la maladie. L'Eculizumab est actuellement le seul traitement autorisé dans l'indication du SHU atypique. Ce traitement est extrêmement coûteux puisqu'un flacon de 300mg d'Eculizumab coûte plus de 4000 euros et qu'en entretien, la posologie est de 1200mg par 14 jours.

Bien que souvent efficaces, ces traitements ont un délai d'action retardé qui est variable entre individus et selon la nature de la MAT. Or, le diagnostic étiologique peut prendre plusieurs jours/semaines. Ainsi, si l'on prend l'exemple du PTT, la mortalité reste actuellement d'environ 15%.

Il existe donc un réel besoin de nouveaux traitements, palliant ces défauts, inconvénients et obstacles de l'art antérieur, en particulier agissant plus rapidement et quelle que soit l'étiologie de la maladie, pour permettre de diminuer la mortalité de la phase aigüe des MAT qui est principalement précoce.

### Description de l'invention

Au terme d'importantes recherches, les inventeurs sont parvenus à résoudre ce problème technique.

De manière surprenante, les inventeurs ont montré que la clusterine se lie aux histones et qu'elle inhibe l'action inflammatoire et cytotoxique de celles-ci.

Les inventeurs ont en effet montré que la clusterine se lie aux histones, molécules de danger au rôle central dans les MAT. Ils ont également montré que la clusterine neutralise les actions des histones, d'une part en bloquant la production de molécules pro-inflammatoires, notamment de cytokines pro-inflammatoires telles que l'IL-6 et le TNFα par les monocytes humains, et d'autre part en bloquant l'induction de la mort des cellules endothéliales, notamment en neutralisant la capacité des histones à induire la mort des cellules endothéliales. Les inventeurs ont également montré que l'ajout de clusterine à des sérums de malades contenant des histones diminue leur toxicité vis-à-vis des cellules endothéliales.

De plus, les inventeurs ont montré que le niveau de clusterine est corrélé à l'état de santé des patients atteints de MAT. Ils ont en effet pu démontrer que les taux de clusterine sérique sont plus bas chez les patients atteints de MAT au diagnostic que chez les sujets sains.

L'invention pourrait faciliter la prise en charge des MAT pour lesquelles les traitements actuels dépendent de l'étiologie. L'utilisation de la clusterine pourrait donc être proposée à tout patient porteur de MAT quelle que soit l'étiologie, permettant un traitement plus rapide non dépendant du diagnostic étiologique.

Ainsi, un premier objet se rapporte à la clusterine pour son utilisation dans le traitement des micro-angiopathies thrombotiques.

La clusterine (Clu), également appelée apolipoprotéine J, est une glycoprotéine hétérodimérique soluble de 80 kDa liée par des ponts disulfures, fortement conservée pendant l'évolution et parmi les mammifères. La clusterine est abondante dans les fluides physiologiques (à des concentrations allant de 100 à 300 µg / ml dans le sérum humain à titre d'exemple) et est induite en réponse à une grande variété de lésions cellulaires et tissulaires. Il est connu que la clusterine a une activité chaperon et est un homologue fonctionnel des petites protéines de choc thermique (HSP) intracellulaires. Elle se lie aux domaines hydrophobes des protéines non natives et les cible pour une internalisation médiée par récepteur et une dégradation lysosomale intracellulaire. Cette fonction permet à la clusterine d'interagir avec un large spectre de molécules, telles que les lipides, les composants du système du complément, les protéines formant les plaques amyloïdes et les immunoglobulines.

Le terme « clusterine » possède, dans le cadre de la présente invention, son sens général, et désigne une glycoprotéine telle que décrite ci-avant. La clusterine peut être une clusterine animale ou une clusterine humaine. Il peut s'agir d'une clusterine choisie dans le groupe comprenant une clusterine plasmatique, une clusterine recombinante et une clusterine synthétique.

La clusterine plasmatique peut être obtenue par purification à partir du plasma, notamment humain, par toute méthode de purification connue de l'homme du métier, par exemple au moyen d'une immunoaffinité, d'un échange cationique, d'un fractionnement du plasma et/ou d'une chromatographie par exclusion de taille.

La clusterine recombinante peut être obtenue par les techniques standards de l'ADN recombinant, bien connues de l'homme du métier. Par exemple, un gène codant une clusterine peut être introduit au moyen d'un vecteur dans le génome d'une espèce productrice, comme une bactérie, une cellule mammifère en culture ou un animal transgénique. Les vecteurs permettant l'introduction, le maintien et l'expression de gènes dans une cellule hôte sont connus de l'homme du métier. Généralement, ils possèdent des séquences indispensables à l'expression du gène introduit, comme les séquences promotrices, les séquences de polyadénylation, et les gènes de sélection. De tels vecteurs peuvent être choisis parmi ceux connus de l'homme du métier, par exemple parmi les adénovirus, les rétrovirus, les plasmides ou les bactériophages, cette liste n'étant pas limitative. Toute cellule de mammifère peut être utilisée comme cellule hôte, c'est-à-dire comme cellule exprimant le gène codant pour la clusterine, par exemple CHO, CHO dhfr- (par exemple CHO DX BII, CHO DG44), CHO Lec13, YB2/0, SP2/0, NSO, 293, BHK, Jurkat, Vero ou COS.

La clusterine synthétique peut être obtenue par toute méthode connue de design de novo de protéines autre que celle utilisée pour la préparation de protéine recombinante, comme par exemple par assemblage sur une matrice.

Quel que soit le mode de préparation de la clusterine, les séquences de clusterine de nombreuses espèces sont connues et peuvent être utilisées dans le cadre de l'invention. Par exemple, la séquence en acides aminés de la clusterine peut être celle de la séquence SEQ ID NO:1.

La clusterine utilisée peut être un variant ayant au moins 70% d'identité avec la séquence SEQ ID NO:1, par exemple 70, 71, 72, 73, 74, 75, 76, 77, 78, 79, 80, 81, 82, 83, 84, 85, 86, 87, 88, 89, 90, 91, 92,93, 94, 95, 96, 97, 98, 99% ou 100% d'identité avec la séquence SEQ ID NO : 1. L'identité de séquence peut être déterminée au moyen de toute technique adaptée connue de l'homme du métier, par exemple au moyen d'un algorithme d'alignement de séquence comme BLAST P. En particulier, un variant de la clusterine peut être un variant dont la fonction est conservée. Il peut s'agir à ce titre d'une clusterine dans laquelle un résidu d'acide aminé donné dans la clusterine naturelle est modifié dans le variant sans altérer ni la conformation ni la fonction globale de la clusterine, y compris, mais sans s'y limiter, le remplacement d'un acide aminé par un acide aminé ayant des propriétés similaires, comme par exemple la polarité, le potentiel de liaison à l'hydrogène, l'hydrophobicité, l'acidité, la basicité, l'aromaticité, etc. En conséquence, un variant désigne également un polypeptide qui a au moins 70% d'identité d'acide aminé et qui a les mêmes propriétés ou fonctions identiques ou substantiellement similaires à la protéine native ou parentale à laquelle il est comparé.

Sans vouloir être lié par un mécanisme d'action, il semble que l'action de la clusterine soit liée à la fonction chaperon des histones de celle-ci.

Tel qu'utilisé ici, le terme "histone" a sa signification générale. Les histones sont de petites protéines basiques à forte teneur en lysine ou arginine et fonctionnent dans l'emballage de l'ADN. Les histones sont très conservées et peuvent être regroupées en cinq grandes classes: H1 / H5, H2A, H2B, H3 et H4 organisés en deux super-classes des histones du noyau (H2A, H2B, H3 Et H4) et les histones de liaison (HI et H5). Dans le cadre de l'invention, une protéine d'histone peut être une histone pleine longueur, un fragment ou une variante de celle-ci. Une variante d'histone peut être modifiée par exemple par la suppression, l'addition et / ou la substitution d'acides aminés. Alternativement, une histone peut être modifiée par acétylation et / ou méthylation de la lysine et de l'arginine. En général, les modifications ne compromettent pas substantiellement la nature polycationique de l'histone ou la capacité de l'histone à se localiser dans un organe.

Avantageusement, la clusterine se lie aux histones de manière à inhiber tout ou partie de l'action inflammatoire et/ou toxique de celles-ci. Il peut s'agir d'une inhibition d'au moins 20%, par exemple 30%, ou 40%, ou 50%, ou 60%, ou 70%, ou 80%, ou 90%, ou 100%, de l'action inflammatoire et/ou toxique des histones.

Avantageusement, la clusterine bloque la production de molécules pro-inflammatoires, notamment de cytokines pro-inflammatoires telles que l'IL-6 et le TNFα par les monocytes humains. Il peut s'agir du blocage d'au moins 20%, par exemple 30%, ou 40%, ou 50%, ou 60%, ou 70%, ou 80%, ou 90%, ou 100%, de la production des molécules pro-inflammatoires.

Avantageusement, la clusterine bloque l'induction de la mort des cellules endothéliales, notamment en neutralisant la capacité des histones à induire la mort des cellules endothéliales. Il peut s'agir du blocage d'au moins 20%, par exemple 30%, ou 40%, ou 50%, ou 60%, ou 70%, ou 80%, ou 90%, ou 100%, de l'induction de la mort des cellules endothéliales.

Dans le cadre de l'invention, tout ou partie des effets cités ci-avant peut avoir lieu.

Les micro-angiopathies thrombotiques peuvent être choisies parmi le purpura thrombotique thrombocytopénique (PTT), le syndrome hémolytique et urémique (SHU) associé ou non à des bactéries, le syndrome de HELLP et des formes secondaires de MAT, par exemple celles survenant lors de certains traitements, lors d'une chimiothérapie, lors de cancers ou lors d'une allogreffe de moelle.

On entend par « traitement », au sens de la présente invention, l'administration de la clusterine à un sujet soit présentant un ou plusieurs symptômes d'une MAT, soit diagnostiqué pour une MAT sans présenter de symptômes de la MAT, par exemple diagnostiqué à un stade précoce de la MAT. Avantageusement, le traitement peut être administré pour traiter toute MAT, c'est-à-dire que ce soit un SHU, un PTT, un syndrome de HELLP ou une MAT secondaire, sans que le type de MAT ait été nécessairement préalablement déterminé. Cela présente l'avantage de pouvoir traiter des patients sans attendre d'avoir défini le sous-type de MAT, et ainsi éviter l'aggravation de la maladie, et/ou l'apparition de symptômes. Alternativement, le traitement peut être administré après dosage de la clusterine, ce dosage permettant de stratifier les patients et éventuellement de ne proposer le traitement qu'aux patients présentant de faibles taux de clusterine, qui est un marqueur de la sévérité de la maladie.

Avantageusement, le traitement peut être une amélioration ou une disparition d'un ou plusieurs symptômes de la MAT, ou une stabilisation de tout ou partie des symptômes de la MAT, ou un ralentissement de la progression de tout ou partie des symptômes de la MAT.

Le traitement peut être réalisé par l'administration d'une dose thérapeutiquement efficace de clusterine, qui est une dose minimale permettant de conférer le bénéfice thérapeutique à un patient. Il peut s'agir d'une dose déterminée par le médecin en fonction de la pathologie, du stade de la pathologie, du dosage initial du niveau de clusterine du patient et/ou du patient. Par exemple, la dose journalière de clusterine peut être comprise entre 0,01 et 1000 mg par adulte par jour. Il peut s'agir par exemple d'une dose journalière de 0,01, 0,05, 0,1, 0,5, 1,0, 2,5, 5,0, 10,0, 15,0, 25,0, 50,0, 100, 250 ou 500 mg de clusterine. Le dosage peut être adapté en fonction du stade de traitement, par exemple être augmenté ou diminué en cours de traitement en fonction des observations du médecin.

Le traitement peut être administré par toute voie d'administration adaptée. Il peut par exemple s'agir d'une administration orale ou d'une administration parentérale de la clusterine. Dans le cas d'une administration parentérale, il peut s'agir par exemple d'une administration intraveineuse, d'une administration intramusculaire ou d'une administration sous-cutanée.

Avantageusement, la clusterine peut être administrée comme décrit ci-avant, en combinaison avec au moins un autre principe actif ou traitement connu dans le traitement des MAT. Dans le cas du PTT, il peut s'agir d'au moins un traitement choisi parmi les échanges plasmatiques, la corticothérapie, les immunosuppresseurs, un anti-CD20 comme par exemple le Rituximab, et un anticorps monoclonal dirigé contre le facteur de von Willebrand comme le Caplacizumab. Dans le cas du SHU typique, il peut s'agir d'au moins un traitement choisi parmi un traitement symptomatique, les échanges plasmatiques, un anticorps monoclonal dirigé contre la fraction C5 du complément comme par exemple l'Eculizumab et un anti-CD20 comme par exemple le Rituximab. Dans le cas du SHU atypique, il peut s'agir d'au moins un traitement choisi parmi les échanges plasmatiques et un anticorps monoclonal dirigé contre la fraction C5 du complément comme l'Eculizumab. Dans le cas du syndrome de HELLP, il peut s'agir d'au moins un traitement choisi parmi la transfusion et le déclenchement de la naissance si l'état de la patiente s'aggrave.

Un autre objet de l'invention se rapporte à une composition pharmaceutique comprenant de la clusterine pour son utilisation dans le traitement des micro-angiopathies thrombotiques, ladite composition ne comprenant pas de protéase du facteur de von Willebrand, ADAMTS13.

Notamment, la composition pharmaceutique de l'invention peut être utilisée dans le traitement du SHU et du syndrome de HELLP.

La clusterine est telle que décrite ci-avant.

La composition pharmaceutique peut comprendre environ 0,01 mg à environ 1000 mg de clusterine, par exemple d'environ 1 mg à environ 100 mg. Une dose effective de clusterine est généralement fournie à une dose d'environ 0,0002 mg/kg à environ 20 mg/kg de poids corporel par jour, plus particulièrement d'environ 0,001 mg/kg à 7 mg/kg de poids corporel par jour.

Typiquement, la composition pharmaceutique peut comprendre, en plus de la clusterine, un ou plusieurs excipients pharmaceutiquement acceptables, et éventuellement au moins une matrice à libération prolongée, telle que des polymères biodégradables.

"Pharmaceutique" et "pharmaceutiquement acceptable" se réfèrent à des entités moléculaires ou des compositions dont la qualité répond aux exigences des normes en vigueur. Un véhicule ou un excipient pharmaceutiquement acceptable se réfère à un agent de remplissage, un diluant, un matériau d'encapsulation ou un adjuvant de formulation semi-solide ou liquide non toxique de tout type adapté. Dans la composition pharmaceutique de la présente invention pour la voie orale, sublinguale, sous-cutanée, administration intramusculaire, intraveineuse, transdermique, locale ou rectale, la clusterine, seule ou en combinaison avec un autre principe actif, peut être administrée dans une forme d'administration unitaire, éventuellement en mélange avec des supports pharmaceutiques conventionnels, à des animaux et êtres humains.

Les formes d'administration unitaires appropriées comprennent des formes à voie orale telles que des comprimés, capsules de gel, poudres, granulés et suspensions ou solutions orales, les formes d'administration sublinguales et buccales, les aérosols, implants, les formes d'administration sous-cutanée, transdermique, topique, intrapéritonéale, intramusculaire, intraveineuse, subdermique, transdermique, intrathécale, intra-artérielle et intranasale, et les formes d'administration rectale. Généralement, la composition pharmaceutique peut contenir des véhicules, qui sont pharmaceutiquement acceptables pour une formulation injectable. Ceux-ci peuvent être en particulier des solutions isotoniques, stériles, salines (comme le phosphate monosodique ou disodique, chlorure de sodium, potassium, calcium ou magnésium et analogues ou des mélanges de tels sels), ou des compositions sèches, en particulier lyophilisées, qui, par addition, selon les cas, d'eau stérilisée ou de solution saline physiologique, permettent la constitution de solutions injectables. Les formes pharmaceutiques adaptées à une utilisation injectable comprennent des solutions aqueuses stériles ou des dispersions, des formulations incluant l'huile de sésame, l'huile d'arachide ou un propylène glycol aqueux, et des poudres stériles pour la préparation extemporanée de solutions ou dispersions injectables stériles. Dans tous les cas, la formulation doit être stérile et fluide pour pouvoir être injectée à l'aide d'une seringue. Elle doit être stable dans les conditions classiques de fabrication et de stockage et doit être stable pour éviter la contamination par des microorganismes, comme les bactéries et les champignons. Des solutions comprenant des composés de l'invention comme base libre ou des sels pharmacologiquement acceptables peuvent être préparés dans l'eau de manière appropriée éventuellement avec un tensioactif, tel que l'hydroxypropylcellulose. Les dispersions peuvent également être préparées en milieu glycérol, polyéthylèneglycols liquides et leurs mélanges et dans les huiles. Aux conditions de stockage et d'utilisation ordinaires, ces préparations peuvent contenir un conservateur pour éviter la croissance de microorganismes. La clusterine peut être formulée sous une forme neutre ou d'un sel.

Les sels pharmaceutiquement acceptables comprennent les sels d'addition d'acide (formés avec les groupes amino libres de la protéine) et qui sont formés avec des acides inorganiques tels que, par exemple, les acides chlorhydrique ou phosphorique, ou des acides organiques tels que les acides acétique, oxalique, tartrique, ou mandélique. Les sels formés avec les groupes carboxyle libres peuvent également être dérivés de bases inorganiques telles que, par exemple, des hydroxydes de sodium, de potassium, d'ammonium, de calcium ou ferriques, et des bases organiques telles que l'isopropylamine, la triméthylamine, l'histidine et la procaïne. Le support peut également être un solvant ou un milieu de dispersion contenant, par exemple, de l'eau, de l'éthanol, du polyol (par exemple, du glycérol, du propylène glycol et du polyéthylène glycol liquide, etc.), des mélanges appropriés de ceux-ci et des huiles végétales. La fluidité appropriée peut être maintenue, par exemple, par l'utilisation d'un revêtement, tel que la lécithine, par le maintien de la taille de particule requise dans le cas de la dispersion et par l'utilisation de tensioactifs. La prévention de l'action des microorganismes peut être réalisée par addition d'agent antibactérien et antifongique, par exemple les parabènes, le chlorobutanol, le phénol, l'acide sorbique ou le thimérosal. Eventuellement, il peut être préférable d'inclure des agents isotoniques, par exemple des sucres ou du chlorure de sodium. L'absorption prolongée des compositions injectables peut être provoquée par l'utilisation dans la composition d'agents retardant l'absorption, par exemple le monostéarate d'aluminium et la gélatine. Des solutions injectables stériles peuvent être préparées en incorporant les composés actifs en quantité requise dans le solvant approprié avec plusieurs des autres ingrédients énumérés ci-dessus, au besoin, suivie d'une stérilisation par filtration. Généralement, les dispersions peuvent être préparées par incorporation des différents ingrédients actifs stérilisés dans un véhicule stérile qui contient le milieu de dispersion basique et les autres ingrédients possibles, énumérés ci-dessus. Dans le cas des poudres stériles pour la préparation de solutions injectables stériles, les procédés de préparation préférées sont des techniques de séchage sous vide et de lyophilisation qui produisent une poudre de l'ingrédient actif plus tout ingrédient désiré supplémentaire à partir d'une solution préalablement stérilisée par filtration de celle-ci. Pour l'administration parentérale dans une solution aqueuse, par exemple, la solution peut être convenablement tamponnée si nécessaire et le diluant liquide peut être d'abord rendu isotonique avec suffisamment de solution saline ou de glucose. Ces solutions aqueuses sont particulièrement appropriées pour les voies intraveineuse, intramusculaire, sous-cutanée et intrapéritonéale.

Un autre objet de l'invention se rapporte à un procédé *ex vivo* de stratification d'un patient atteint de MAT ou susceptible de l'être, comprenant les étapes suivantes:
1) Mesure, dans un échantillon biologique dudit patient, du taux Le de clusterine, et
2) Comparaison du taux L_{c} mesuré à l'étape 1) avec un taux de clusterine de référence L_{ref} par calcul du score S1= L_{c}/L_{ref},
dans lequel :
- Si S1 ≤ 1, le patient est considéré comme susceptible de recevoir un bénéfice d'un traitement de la MAT par la clusterine,
- Si S1 > 1, le patient n'est pas considéré comme susceptible de recevoir un bénéfice d'un traitement de la MAT par la clusterine.

On entend par « stratification », au sens de la présente invention, l'approche thérapeutique où l'objectif est de sélectionner les patients auxquels administrer un traitement en fonction d'un marqueur prédictif, en l'occurrence la clusterine, afin de ne traiter que la sous-population pour laquelle le traitement est susceptible d'apporter un bénéfice parmi ceux diagnostiqués pour une MAT.

Le procédé de stratification de l'invention peut donc être réalisé en amont d'un traitement par la clusterine à des patients diagnostiqués pour une MAT, afin de cibler une population la plus susceptible de recevoir un bénéfice du traitement. En effet, les inventeurs ont montré que les patients qui présentent des taux bas de clusterine lors du diagnostic de la MAT, notamment inférieurs ou égal à 200 µg/mL, ont également des taux de plaquettes plus bas et des taux de LDH plus élevés, qui sont deux marqueurs de la sévérité, au moins hématologique, de la maladie.

On entend par « échantillon biologique », au sens de la présente invention, tout échantillon biologique obtenu à partir d'un sujet, en particulier déjà diagnostiqué ou en cours de diagnostic pour une MAT. L'échantillon doit être susceptible de contenir de la clusterine. L'échantillon peut être par exemple un fluide corporel, pouvant ou non contenir des cellules, par exemple du sang, que ce soit du sérum et/ou du plasma, de l'urine, de la salive ou une biopsie. Il peut également s'agir d'une coupe de tissu telle que des coupes congelées prises à des fins histologiques. Le terme "échantillon biologique" englobe également tout matériau dérivé d'un échantillon biologique, par exemple les cellules (ou leur descendance) isolées de l'échantillon, ou des protéines extraites de l'échantillon. Le traitement d'un échantillon biologique peut impliquer une ou plusieurs étapes choisies parmi la filtration, la distillation, l'extraction, la concentration, l'inactivation de composants interférants ou l'addition de réactifs. Lorsque l'échantillon biologique est un échantillon de sang, il peut s'agir de sang total, de sérum ou de plasma.

L'étape de mesure, dans l'échantillon biologique du patient, du taux L_{c} de clusterine, peut être réalisée par tout procédé adapté connu de l'homme du métier. La clusterine mesurée peut être de la clusterine libre ou de la clusterine complexée. Cette étape peut par exemple comprendre la mise en contact de l'échantillon avec un ou plusieurs partenaires de liaison capables d'interagir sélectivement avec la clusterine contenue dans l'échantillon biologique. Un partenaire de liaison peut être un anticorps, comme des anticorps monoclonaux ou des aptamères. Des supports solides pouvant être utilisés dans la mise en œuvre de cette étape peuvent être par exemple des substrats tels que la nitrocellulose (par exemple, en forme de membrane ou de microtitrage), le chlorure de polyvinyle (par exemple, les feuilles ou les puits de microtitrage), le latex de polystyrène (par exemple, perles ou plaques de microtitrage), le fluorure de polyvinylidine, le papier diazoté, les membranes de nylon, les billes activées, ou les billes magnétiquement réactives, cette liste n'étant pas limitative. La clusterine peut ensuite être révélée avec un second partenaire de liaison, comme un anticorps ou un aptamère spécifique de la clusterine. En règle générale, le second partenaire de liaison est un anticorps spécifique conjugué à une enzyme. Le niveau de clusterine peut être mesuré en utilisant des techniques d'immunodiagnostic standard, y compris des immunodosages tels que la compétition, la réaction directe ou les essais de type sandwich. De tels essais comprennent sans y être limités à des tests d'agglutination, des immunodosages médiés par des enzymes, tels que des ELISA, des dosages du type biotine / avidine, les radioimmunodosages, l'immunoélectrophorèse ou l'immunoprécipitation. Il peut s'agir par exemple du test de dosage Human Clusterin ELISA Kit, Thermo Scientific™ Pierce™ commercialisé par Invitrogen ou un test Quantikine ® ELISAs - R&D Systems, spécifique du dosage dans le sérum et le plasma humain.

La valeur de référence prédéterminée L_{ref} peut être relative à une valeur dérivée d'études de population, incluant, sans limitation, des sujets de même tranche d'âge ou d'une tranche d'âge similaire, des sujets appartenant au même groupe ethnique ou à un groupe ethnique similaire, et des sujets présentant un même degré de sévérité de la maladie. De telles valeurs de référence prédéterminées peuvent être dérivées d'analyses statistiques et / ou de données de prédiction des risques des populations obtenues à partir d'algorithmes mathématiques et d'indices calculés pour la maladie. Par exemple, la valeur de référence prédéterminée peut être dérivée du niveau de clusterine dans un échantillon témoin issu d'un ou plusieurs sujets non atteints de MAT. Alternativement, une mesure rétrospective du niveau de clusterine d'échantillons de sujets historiquement correctement classés peut être utilisée pour établir la valeur de référence prédéterminée.

On entend par « bénéfice d'un traitement de la MAT », au sens de la présente invention, une amélioration ou une disparition d'un ou plusieurs symptômes de la MAT, ou une stabilisation de tout ou partie des symptômes de la MAT, ou un ralentissement de la progression de tout ou partie des symptômes de la MAT.

Par exemple, pour la détermination de la valeur de référence, le niveau de clusterine peut être mesuré dans 100 échantillons biologiques de 100 sujets. Une fois les deux premiers sous-ensembles séparés, des courbes de Kaplan Meier sont réalisées pour chacun des deux sous-ensembles, et la valeur p entre les deux sous-ensembles peut être calculée. La valeur de référence est ensuite sélectionnée de manière telle que la discrimination sur la base du critère de la valeur p minimale est la plus forte. En d'autres termes, le niveau de clusterine correspondant à la limite entre les deux sous-ensembles pour lequel la valeur p est minimale est considéré comme la valeur de référence prédéterminée. Il convient de noter que la valeur de référence prédéterminée n'est pas nécessairement la valeur médiane du niveau de clusterine.

Ainsi, la valeur de référence prédéterminée permet une discrimination entre un patient pouvant être considéré comme susceptible de recevoir un bénéfice d'un traitement de la MAT par la clusterine, et un patient pouvant être considéré comme non susceptible de recevoir un bénéfice d'un traitement de la MAT par la clusterine.

D'autres avantages pourront encore apparaître à l'homme du métier à la lecture des exemples ci-dessous, illustrés par les figures annexées, donnés à titre illustratif.

### Brève description des figures

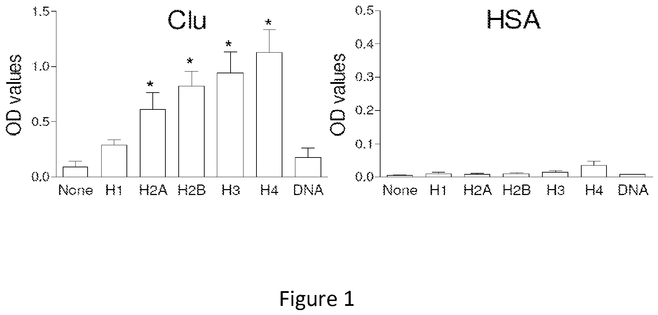

- La figure 1 représente la liaison de la clusterine aux histones. Les histones H1, H2A, H2B, H3 et H4 (New England Biolabs, Ipswich, MA) ou de l'ADN génomique issu de cellules humaines, ont été immobilisées au fond de plaques ELISA (1 µg/ml) en tampon carbonate/bicarbonate 10 mM, pH=9.6. Après saturation, les plaques ont été incubées avec 1 µg/ml de clusterine humaine recombinante (R&D systems, Abingdon, UK) ou de sérum albumine humaine (HSA ; Sigma, St Louis, MO), couplées à la biotine. La fixation de Clu ou de HSA aux histones a été mise en évidence avec de la streptavidine couplée à la peroxidase. Les résultats sont exprimés en densité optique (moyenne ± SD, n=5). La molécule HSA a été utilisée comme protéine contrôle. La fixation de l'ADN génomique dans les plaques ELISA a été vérifiée avec un anticorps anti-ADN couplé à la peroxidase (données non présentées).

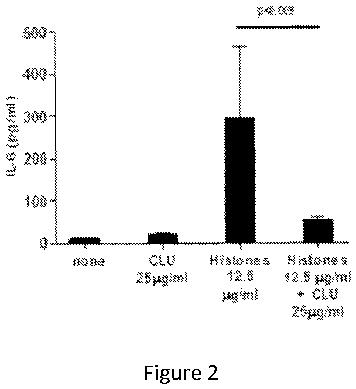

- La figure 2 représente la quantification par ELISA de l'IL-6, en pg/ml, dans les surnageants de culture de monocytes humains isolés du sang de donneurs sains par tri magnétique puis incubés pendant 12 h avec 12.5 µg/ml d'histones, en présence (diagramme 4) ou en absence (diagramme 3) de 25 µg/ml de clusterine (CLU). Le diagramme 1 est le témoin de culture des monocytes seuls, et le diagramme 2 est le témoin de culture des monocytes incubés avec 25 µg/ml de clusterine.

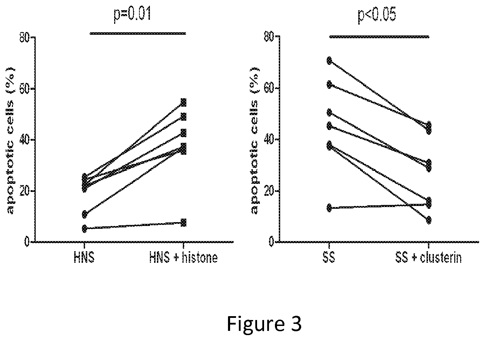

- La figure 3 représente le pourcentage de cellules apoptotiques mesuré dans le cas de cellules endothéliales humaines incubées pendant 15 min soit (1) graphique de gauche : en présence de sérums contrôles (HNS) en présence ou non d'histones (50 ng/ml) Histones de chez Roche), soit (2) graphique de droite : avec des sérums de patients contenant des histones endogènes (SS ; n=5, 10% vol/vol) en présence ou non de 200 ng/ml de Clu.

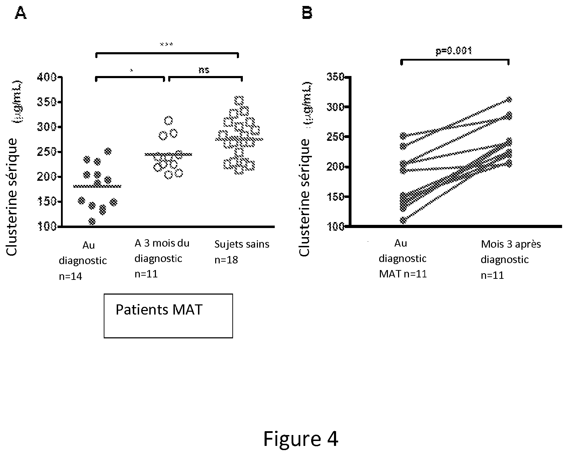

- La figure 4 représente le dosage de la clusterine sérique, en µg/ml, chez des patients atteints de MAT et des patients sains. (A) La clusterine sérique est mesurée chez des patients sains (carrés) et des patients MAT soit au début de la MAT (cercles pleins) soit 3 mois après le diagnostic (cercles vides). (B) L'évolution de la clusterine sérique chez des patients MAT au diagnostic (points de gauche) et 3 mois après le diagnostic (points de droite). Chaque point représente un patient. L'analyse statistique a été réalisée au moyen de tests non paramétriques.

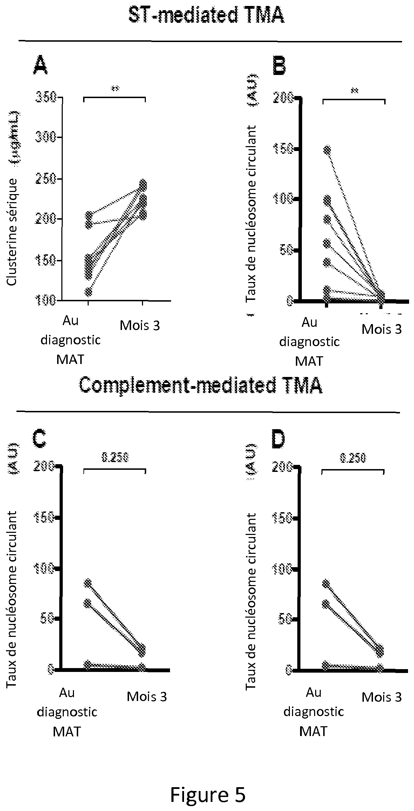

- La figure 5 représente (A) pour les patients atteints de SHU typique, le dosage de clusterine sérique en µg/ml, au diagnostic de la MAT (gauche) et après 3 mois de traitement (droite) par Eculizumab (SOLIRIS) ; (B) pour les patients atteints de SHU typique, le taux de nucléosomes circulants (AU) au diagnostic de la MAT (gauche) et après 3 mois de traitement (droite) par Eculizumab (SOLIRIS). Les patients ST mediated-TMA (SHU typiques) ont été traités de façon homogène par Eculizumab (SOLIRIS) selon le même schéma de traitement. (C) et (D) pour les patients atteints de MAT médiée par le complément, le taux de nucléosomes circulants (AU) au diagnostic de la MAT (gauche) et après 3 mois de traitement (droite) par Eculizumab (SOLIRIS) ; les patients complement-mediated TMA ont été traités de façon variable selon le contexte clinique.

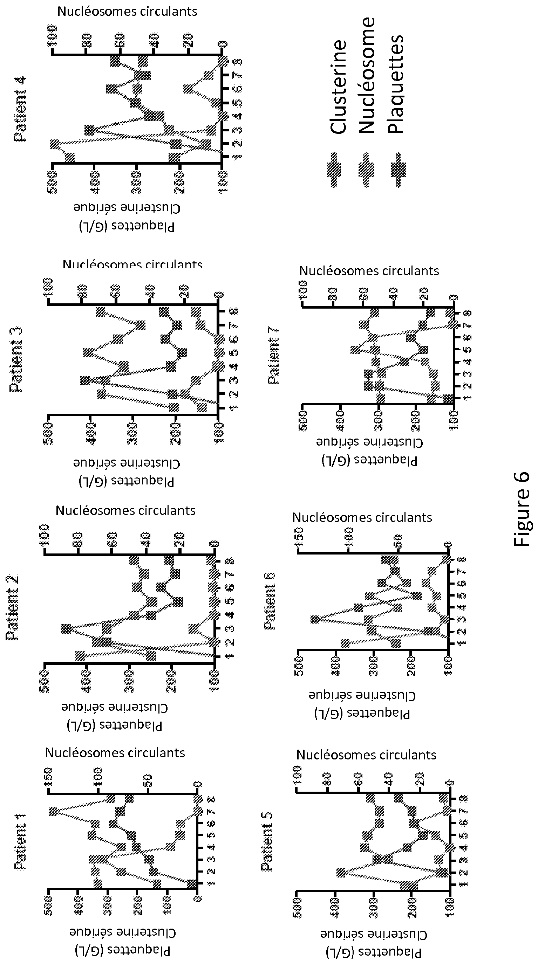

- La figure 6 représente la concentration en plaquettes, en g/l (gris foncé), en clusterine sérique en µg/ml (gris clair) et en nucléosomes circulants en UA (gris moyen), chez 7 patients atteints de MAT, de la 1^{ère} à la 8^{ème} injection d'Eculizumab des patients (tHUS, n = 7). La clusterine sérique, les nucléosomes circulants et la concentration plaquettaire ont été mesurés juste avant chaque injection d'Eculizumab. Eculizumab était injecté à répétition toutes les 2 semaines. La 8ème injection s'est produite à un délai moyen de 2 mois à partir du diagnostic d'aHUS.

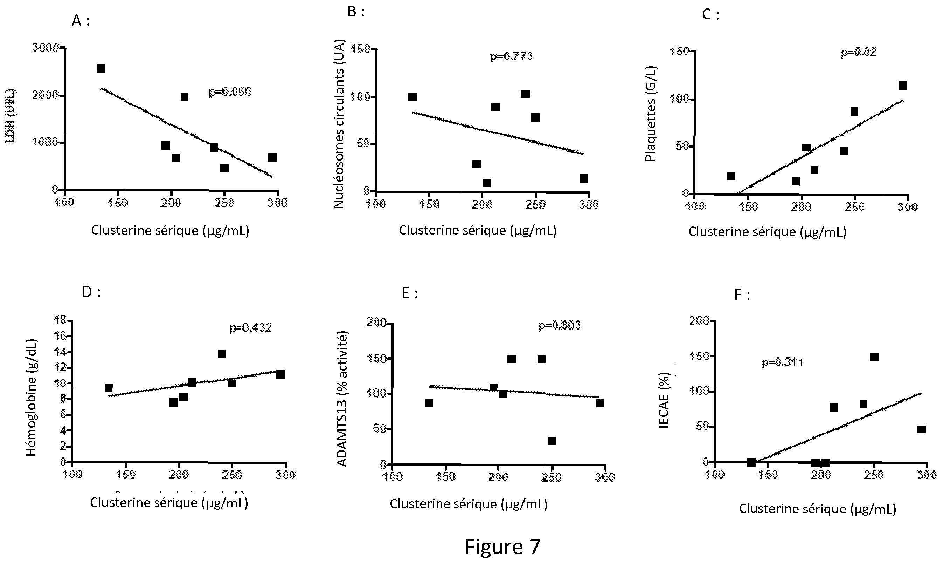

- La figure 7 représente A : la concentration de LDH (UI/ml), B : la concentration en nucléosomes circulants (UA), C: la concentration en plaquettes (g/l), D : la concentration en hémoglobine (g/dl), E : la concentration en ADAMTS13 (% d'activité) et F : le pourcentage de IECAE (une unité arbitraire permettant de mesurer l'activité fonctionnelle de la voie terminale commune du complément par test ELISA, société Diasorin) en fonction de la concentration en clusterine sérique (µg/ml) chez un patient au moment du diagnostic d'une tHUS (HUS médiée par la shiga toxine).

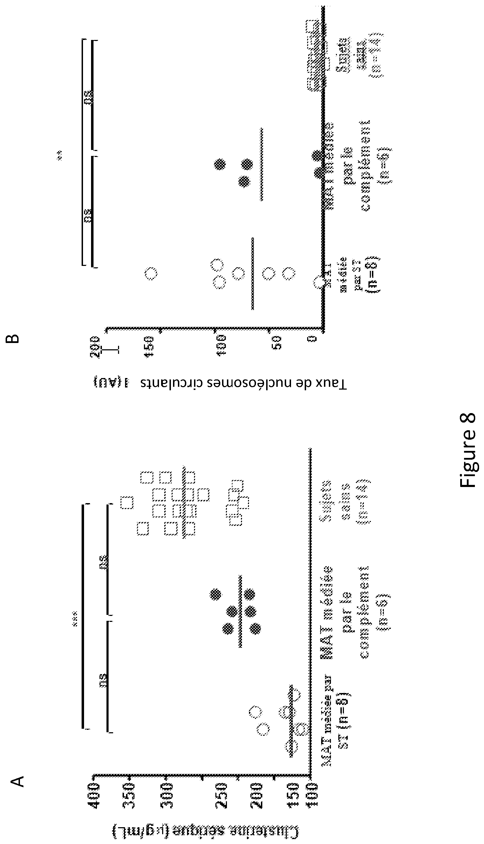

- La figure 8 représente (A): le dosage de la clusterine sérique, en µg/ml, chez des patients atteints de MAT médiée par la shiga toxine tHUS (n=8), des patients atteints de MAT médiée par le complément aHUS (n=6) et des sujets sains (n=14) ; (B) le taux de nucléosomes circulants, en AU, chez des patients atteints de MAT médiée par la shiga toxine (n=8), des patients atteints de MAT médiée par le complément (n=6) et des sujets sains (n=14).

### EXEMPLES

### Exemple 1 : La clusterine neutralise les actions des histones en bloquant la production de molécules pro-inflammatoires

Des monocytes humains ont été isolés du sang de donneurs sains par tri magnétique (tri positif sur la base de l'expression du marqueur CD14; Mylteny Biotech, Bergish Gladbach, Germany). Les monocytes (2x106 cellules/ml) ont été incubés pendant 12 h avec 12,5 µg/ml d'histones (Sigma), en présence ou en absence de 25 µg/ml de clusterine (CLU). L'IL-6 a été quantifiée par ELISA (Diaclone, Besançon, France) dans les surnageants de culture. Les résultats sont exprimés en pg/ml (moyenne ± SD, n=6).

Les résultats sont montrés en Figure 2.

### Exemple 2 : La clusterine protège les cellules endothéliales de la mort induite par des sérums contenant des histones.

Des cellules endothéliales humaines (HDMEC de chez Promocell) sont incubées pendant 15 min soit en présence de sérums contrôles (HNS ; idem) en présence ou non d'histones (50 ng/ml Histones de chez Roche), soit avec des sérums de patients contenant des histones endogènes (SS ; n=5, 10% vol/vol) en présence ou non de 200 ng/ml de Clu. Après 15 min, les cellules endothéliales sont marquées avec de l'annexine V et de l'iodure de propidium (Kit Becton Dickinson) afin d'évaluer le pourcentage de cellules apoptotiques par cytométrie en flux.

Les résultats sont montrés en Figure 3.

### Exemple 3 : Le niveau de clusterine est associé à l'activité de la MAT

Les taux de clusterine sérique sont plus bas chez les patients atteints de MAT (TMA) au diagnostic que chez les sujets sains (healthy subjects). Le taux de clusterine sérique a été dosé chez 11/14 patients au 3ieme mois du diagnostic de MAT, après traitement. Il ne différait pas significativement de celui de la population de sujets sains, mais était significativement plus élevé qu'au diagnostic. La clusterine a été dosée par ELISA (R&D Systems) dans des sérums de sujets sains et de patients atteints de MAT.

Les résultats sont montrés en Figure 4.

### Exemple 4: Le déficit en clusterine est observé dans différents types de MAT

Un dosage de la clusterine, des histones et d'un marqueur d'activation de la voie terminale commune dans le sérum est effectué chez des patients atteints d'un SHU typique (20 cas) ou atypique (10 cas), d'un PTT (30 cas) et/ou du syndrome de HELP (50 cas), au diagnostic et en suivi. Ces dosages sont réalisés par des techniques ELISA commerciales.

Les résultats attendus permettent d'établir des associations statistiques et/ou des corrélations avec les principaux critères biologiques de suivi des patients (taux de plaquettes, LDH, haptoglobine, marqueurs d'atteinte rénale (créatininémie, rapport albuminurie/créatininurie, etc..) ou des marqueurs d'atteinte extra-rénale si applicable (troponine, ASAT/ALAT, gammaGT...).

### Exemple 5 : La clusterine est un cytoprotecteur endothélial dans les syndromes de MAT

Des cellules endothéliales microvasculaires sont incubées en présence de sérums de patients affectés de SHU, de PTT ou du syndrome de HELLP avec ou sans ajout de clusterine recombinante (dose cible 25 µg/mL) pendant 2h, 6h ou 24h. L'analyse de la mort cellulaire (apoptotique ou nécrotique) est réalisée par marquage en cytométrie de flux (annexine-5, iodure de propidium).

Il est attendu que les histones et nucléosomes circulants dans les sérums des patients atteints de MAT engendrent des lésions endothéliales, et que l'addition de clusterine protège les cellules endothéliales de la mort induite par ces éléments toxiques.

### Exemple 6 : La clusterine démontre un effet in vivo dans un modèle murin de SHU typique

Deux types d'analyses sont réalisés.
1) Etude de la toxicité rénale induite par la STX dans des souris C57BL/6J invalidées pour le gène de la clusterine (KO Clu) pour mettre en évidence une aggravation de la toxicité rénale et des marqueurs biologiques de SHU (thrombopénie, anémie) en l'absence de clusterine Dans une première partie des expériences, une dose sub-létale est recherchée afin de suivre plus précisément les marqueurs du SHU que sont l'anémie, la thrombocytopénie et l'insuffisance rénale. Les doses de 625 pg/g, 300 pg/g et 100 pg/g sont testées sur des animaux sauvages (10 souris par dose, plus 10 souris contrôles). Les souris sont pesées quotidiennement, le poids est utilisé comme indicateur de toxicité car la perte de poids est corrélée dans le modèle murin avec la maladie.
   Une fois la dose établie, les souris WT et les souris clusterine-KO sont injectées par voie intra-péritonéale par la dose choisie de STX ou par du PBS et sont ensuite suivies par prélèvement de sang sur lequel une numération formule sanguine est faite. L'hémoglobine est dosée en parallèle afin de calculer un index d'anémie. Le sérum est aussi analysé pour les marqueurs d'insuffisance rénale (créatinine et cystatine). Les reins sont prélevés pour analyser les dépôts de protéine C3, marqueur de lésions rénales.
2) Etude du rôle protecteur et thérapeutique de la clusterine dans la MAT dans un modèle murin de SHU dans les souris C57BL/6J normales (non déficientes en clusterine)

Les souris WT sont injectées par voie intra-péritonéale par la dose choisie de STX ou par du PBS. Les souris sont traitées par injections régulières de clusterine murine recombinante (1 mg/kg). Le suivi est effectué par prélèvement de sang sur lequel une numération formule sanguine est faite. L'hémoglobine est dosée en parallèle afin de calculer un index d'anémie. Le sérum est aussi analysé pour les marqueurs d'insuffisance rénale (créatinine et cystatine). Les reins sont prélevés pour analyser les dépôts de protéine C3, marqueur de lésions rénales.

Il est attendu que :
- il existe une corrélation entre l'absence de clusterine et l'aggravation de la toxicité rénale et des marqueurs biologiques de SHU (thrombopénie, anémie)
- les résultats permettent de démontrer le rôle non redondant de la clusterine pour contrôler une toxicité induite par shiga toxine dans un modèle murin
- les résultats démontrent un rôle protecteur et thérapeutique de la clusterine dans la MAT dans un modèle murin de SHU et mettent en évidence une amélioration de la fonction rénale et des paramètres d'hémolyse et de thrombopénie des souris supplémentées par rapport au groupe contrôle.

## Revendications

1. Clusterine pour son utilisation dans le traitement des micro-angiopathies thrombotiques.

2. Clusterine pour son utilisation selon la revendication 1, ladite clusterine étant une clusterine humaine de séquence SEQ ID NO : 1.

3. Clusterine pour son utilisation selon la revendication 1, ladite clusterine ayant une séquence peptidique présentant au moins 70%, de préférence au moins 80% d'identité avec la séquence SEQ ID NO : 1.

4. Clusterine pour son utilisation selon l'une quelconque des revendications précédentes, ladite clusterine étant choisie parmi une clusterine recombinante, une clusterine plasmatique et une clusterine synthétique.

5. Clusterine pour son utilisation selon l'une quelconque des revendications précédentes, dans laquelle les micro-angiopathies thrombotiques sont choisies parmi le purpura thrombotique thrombocytopénique (PTT), le syndrome hémolytique et urémique (SHU) associé ou non à des bactéries, le syndrome de HELLP et une MAT secondaire.

6. Clusterine pour son utilisation selon l'une quelconque des revendications précédentes, dans laquelle ledit traitement comprend une administration orale ou parentérale de ladite clusterine.

7. Clusterine pour son utilisation selon la revendication 6, dans laquelle ladite administration parentérale est sélectionnée dans le groupe comprenant une administration intraveineuse, une administration intramusculaire et une administration subcutanée.

8. Composition pharmaceutique comprenant de la clusterine pour son utilisation dans le traitement des micro-angiopathies thrombotiques, ladite composition ne comprenant pas de protéase du facteur de von Willebrand.

9. Composition pharmaceutique pour son utilisation selon la revendication 8, dans laquelle ladite clusterine est telle que définie dans l'une quelconque des revendications 2 à 4.

10. Composition pharmaceutique pour son utilisation selon la revendication 8 ou 9, ladite composition pharmaceutique étant sous la forme d'une solution injectable.

11. Procédé *ex vivo* de stratification d'un patient atteint de MAT ou susceptible de l'être, comprenant les étapes suivantes:
1) Mesurer, dans un échantillon biologique dudit patient, le taux Le de clusterine, et
2) Comparaison du taux L_{c} mesuré à l'étape 1) avec un taux de clusterine L_{ref} par calcul du score S1= L_{c}/L_{ref},
dans lequel :
• Si S1 ≤ 1, le patient est considéré comme susceptible de recevoir un bénéfice d'un traitement de la MAT par la clusterine,
• Si S1 > 1, le patient n'est pas considéré comme susceptible de recevoir un bénéfice d'un traitement de la MAT par la clusterine.

## Patentansprüche

1. Clusterin zur Verwendung bei der Behandlung von thrombotischen Mikroangiopathien.

2. Clusterin zur Verwendung nach Anspruch 1, wobei das Clusterin ein menschliches Clusterin der Sequenz SEQ ID NO: 1 ist.

3. Clusterin zur Verwendung nach Anspruch 1, wobei das Clusterin eine Peptidsequenz mit mindestens 70%, vorzugsweise mindestens 80% Identität mit der Sequenz SEQ ID NO: 1 aufweist.

4. Clusterin zur Verwendung nach einem der vorhergehenden Ansprüche, wobei das Clusterin ausgewählt ist aus einem rekombinanten Clusterin, einem Plasma-Clusterin und einem synthetischen Clusterin.

5. Clusterin zur Verwendung nach einem der vorhergehenden Ansprüche, wobei die thrombotischen Mikroangiopathien ausgewählt sind aus thrombotischer thrombozytopenischer Purpura (TTP), bakteriellem oder nichtbakteriellem assoziiertem hämolytisch-urämischem Syndrom (HUS), HELLP-Syndrom und sekundärem MAT.

6. Clusterin zur Verwendung nach einem der vorhergehenden Ansprüche, wobei die Behandlung die orale oder parenterale Verabreichung des Clusterins umfasst.

7. Clusterin zur Verwendung nach Anspruch 6, wobei die parenterale Verabreichung ausgewählt ist aus der Gruppe bestehend aus intravenöser Verabreichung, intramuskulärer Verabreichung und subkutaner Verabreichung.

8. Clusterin enthaltende pharmazeutische Zusammensetzung zur Verwendung bei der Behandlung von thrombotischen Mikroangiopathien, wobei die Zusammensetzung keine von Willebrand-Faktor-Protease enthält.

9. Pharmazeutische Zusammensetzung zur Verwendung nach Anspruch 8, wobei das Clusterin wie in einem der Ansprüche 2 bis 4 definiert ist.

10. Pharmazeutische Zusammensetzung zur Verwendung nach Anspruch 8 oder 9, wobei die pharmazeutische Zusammensetzung in Form einer injizierbaren Lösung vorliegt.

11. Ex-vivo-Verfahren zur Stratifizierung eines Patienten mit oder empfänglich für TMA, umfassend die folgenden Schritte:
1) Messen des Lc-Spiegels von Clusterin in einer biologischen Probe des Patienten, und
2) Vergleich der in Schritt 1) gemessenen Rate Lc mit einer Clusterin-Rate Lref durch Berechnung der Punktzahl S1 = Lc/Lref,
Wobei:
• - Wenn S1 ≤ 1 ist, wird der Patient als wahrscheinlich von einer Clusterin-Behandlung von MAT profitieren,
• - Wenn S1 > 1 ist, gilt es als unwahrscheinlich, dass der Patient von einer Clusterin-Behandlung für TAM profitiert.

## Claims

1. Clusterin for use thereof in the treatment of thrombotic microangiopathies.

2. Clusterin for use thereof as claimed in claim 1, said clusterin being a human clusterin of sequence SEQ ID No.: 1.

3. Clusterin for use thereof as claimed in claim 1, said clusterin having a peptide sequence which has at least 70%, preferably at least 80%, identity with the sequence SEQ ID No.: 1.

4. Clusterin for use thereof as claimed in any one of the preceding claims, said clusterin being chosen from a recombinant clusterin, a plasma clusterin and a synthetic clusterin.

5. Clusterin for use thereof as claimed in any one of the preceding claims, wherein the thrombotic microangiopathies are chosen from thrombotic thrombocytopenic purpura (TTP), hemolytic uremic syndrome (HUS) associated or not associated with bacteria, HELLP syndrome and a secondary TMA.

6. Clusterin for use thereof as claimed in any one of the preceding claims, wherein said treatment comprises oral or parenteral administration of said clusterin.

7. Clusterin for use thereof as claimed in claim 6, wherein said parenteral administration is selected from the group comprising intravenous administration, intramuscular administration and subcutaneous administration.

8. A pharmaceutical composition comprising clusterin, for use thereof in the treatment of thrombotic microangiopathies, said composition not comprising von Willebrand factor of protease.

9. The pharmaceutical composition for use thereof as claimed in claim 8, wherein said clusterin is as defined in any one of claims 2 to 4.

10. The pharmaceutical composition for use thereof as claimed in claim 8 or 9, said pharmaceutical composition being in the form of an injectable solution.

11. An *ex vivo* method for stratification of a patient who is or who may be suffering from TMA, comprising the following steps:
1) measuring, in a biological sample from said patient, the clusterin level Lc, and
2) comparing the level Lc measured in step 1) with a clusterin level L_{ref} by calculating the score S1 = L_{c}/L_{ref},
wherein:
• if S1 ≤ 1, the patient is considered to be liable to receive a benefit from a TMA treatment with clusterin,
• if S1 > 1, the patent is not considered to be liable to receive a benefit from a TMA treatment with clusterin.
